(19) Europäisches Patentamt
European Patent Office
Office européen des brevets

(11) **EP 3 439 554 B1**

(12) **EUROPÄISCHE PATENTSCHRIFT**

(45) Veröffentlichungstag und Bekanntmachung des Hinweises auf die Patenterteilung:
**28.10.2020   Patentblatt 2020/44**

(21) Anmeldenummer: **17716836.6**

(22) Anmeldetag: **06.04.2017**

(51) Int Cl.:
*A61B 5/11* (2006.01)     *A43B 3/00* (2006.01)
*A61B 5/00* (2006.01)     *G01N 19/00* (2006.01)
*A61B 5/103* (2006.01)

(86) Internationale Anmeldenummer:
**PCT/EP2017/058264**

(87) Internationale Veröffentlichungsnummer:
**WO 2017/174729 (12.10.2017 Gazette 2017/41)**

(54) **VERFAHREN UND VORRICHTUNG ZUR BESTIMMUNG EINES UNTERGRUNDES**

METHOD AND DEVICE FOR DETERMINING A GROUND TYPE

PROCÉDÉ ET DISPOSITIF DE DÉTERMINATION D'UN SOL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **07.04.2016   DE 102016205812**

(43) Veröffentlichungstag der Anmeldung:
**13.02.2019   Patentblatt 2019/07**

(73) Patentinhaber: **WAEWAE TECH LIMITED**
**Auckland 1010 (NZ)**

(72) Erfinder: **MATTHIES, Denys**
**18055 Rostock (DE)**

(74) Vertreter: **Patentanwälte Bressel und Partner mbB**
**Potsdamer Platz 10**
**10785 Berlin (DE)**

(56) Entgegenhaltungen:
**DE-A1-102014 212 535     US-A1- 2005 183 292**
**US-A1- 2014 174 205**

• **CHENG JINGYUAN ET AL: "Designing Sensitive Wearable Capacitive Sensors for Activity Recognition", IEEE SENSORS JOURNAL, IEEE SERVICE CENTER, NEW YORK, NY, US, Bd. 13, Nr. 10, 1. Oktober 2013 (2013-10-01), Seiten 3935-3947, XP011525582, ISSN: 1530-437X, DOI: 10.1109/JSEN.2013.2259693 [gefunden am 2013-08-30] in der Anmeldung erwähnt**

**Beschreibung**

Die Erkennung eines Untergrundes für Fußgänger stellt für einige technische Anwendungsgebiete eine interessante Problemstellung dar. Auch die Identifizierung eines Nutzers und die Bestimmung einer Haltung des Nutzers ist für bestimmte Anwendungen interessant.

Bekannt ist die Bestimmung von einer Festigkeit eines Untergrunds mittels am Fuß getragener Beschleunigungssensoren. Dies ist z.B. in der WO 2014/032181 beschrieben.

Weiter beschreibt die KR 1020090036732 A Schuhe zur Detektion und Warnung vor spannungsführenden Böden.

Das Dokument "Cheng et al., Designing sensitive wearable capacitive sensors for activity recognition. In Sensors Journal, 13(10), 3935-3947, IEEE, 2013, October" beschreibt die Detektion eines Unterschieds zwischen Treppensteigen, Gehen auf dem Rasen und Gehen auf Asphalt durch die Nutzung von zwei Elektroden am Fuß durch eine kapazitive Sensierung. Das Dokument offenbart ein Sensorelektrodenband, welches um einen Knöchel herum getragen wird. Sensorsignale wurden beim Treppensteigen und beim Gehen auf den Untergründen Rasen und Asphalt erfasst.

Die US 2014/0135954 A1 offenbart einen Schuh mit Näherungs- und Kraftsensoren, wobei diese kapazitive Sensoren sein können.

Die WO 2009/089406 A2 offenbart eine Einlage, die dynamisch Druckverteilungskarten von einem individuellen Fuß erzeugt.

Die US 2005/183292 A1 offenbart intelligente Systeme für Fußbekleidungsartikel.

Es stellt sich das technische Problem, ein Verfahren und eine Vorrichtung zur Bestimmung einer Vielzahl von verschiedener Untergründe für einen Fußgänger zu schaffen, die eine zuverlässige und möglichst genaue Bestimmung des Untergrunds ermöglichen. Die Lösung des technischen Problems ergibt sich durch die Gegenstände mit den Merkmalen der Ansprüche 1 und 8. Weitere vorteilhafte Ausgestaltungen der Erfindung ergeben sich aus den Unteransprüchen.

Vorgeschlagen wird ein Verfahren zur Bestimmung eines Untergrunds. Das Verfahren dient hierbei zur Bestimmung einer Art und/oder mindestens Eigenschaft des Untergrunds und somit auch zur Identifikation eines Untergrunds. Verschiedene Arten von Untergründen können beispielsweise verschiedene Materialarten bezeichnen, z.B. Asphalt, Rasen, Holz, Stein, Linoleum, Sand, Tartan, Teppich, Eis usw. Eigenschaften des Untergrundes können beispielsweise eine elektrische Eigenschaft, z.B. eine Leitfähigkeit, eine mechanische Eigenschaft, z.B. eine Festigkeit oder eine weitere physikalische Eigenschaft sein. Das Verfahren dient somit zur Erkennung und Identifikation eines Untergrunds.

In dem Verfahren wird mindestens ein Kapazitätswert mindestens einer Elektrode bestimmt, wobei die Elektrode im Bereich einer Schuhsohle eines Schuhs angeordnet ist. Hierbei kann die Elektrode insbesondere derart im Bereich der Schuhsohle angeordnet sein, dass die Elektrode weder den Untergrund noch einen Fuß eines Nutzers elektrisch leitend kontaktiert. Mit anderen Worten können zwischen dem Untergrund und der Elektrode sowie zwischen der Elektrode und einer im Schuh angeordneten Fußsohle des Nutzers jeweils mindestens eine Isolierschicht angeordnet sein.

Der Kapazitätswert ergibt sich hierbei in Abhängigkeit verschiedener Kapazitäten, insbesondere in Abhängigkeit einer Kapazität, die zwischen der Elektrode und dem Untergrund gebildet wird und/oder in Abhängigkeit einer Kapazität, die zwischen der Fußsohle des Nutzers und der Elektrode gebildet wird.

Weiter wird der Untergrund in Abhängigkeit des Kapazitätswertes oder eines zeitlichen Verlaufs des Kapazitätswertes bestimmt.

Der Kapazitätswert der mindestens einen Elektrode kann hierbei implizit Informationen über eine Schrittlänge, ein Gewicht, eine Fußform und -größe und über eine AbrollBewegung umfassen.

Der Kapazitätswert kann hierbei aktiv bestimmt werden, wobei die mindestens eine Elektrode Teil eines sogenannten aktiven kapazitiven Sensors ist. Selbstverständlich kann der Kapazitätswert auch passiv bestimmt werden, wobei die Elektrode Teil eines passiven Elektrischen-Feld-Sensors ist.

Insbesondere kann ein Kapazitätswert bestimmt werden, indem eine Ladezeit eines Kondensators bestimmt wird, der die Elektrode umfasst. Der Kondensator kann beispielsweise ein Kondensator sein, der zwischen der Elektrode und dem menschlichen Körper gebildet wird. Auch kann der Kondensator ein Kondensator sein, der zwischen der Elektrode und dem Untergrund gebildet wird. Die Elektroden können hierbei in verschiedenen Betriebsmodi betrieben werden, insbesondere in einem sogenannten Transmit-Modus, in einem Shunt-Modus und/oder in einem Lademodus. Diese Modi sind beispielsweise in dem Dokument "J. R. Smith, Electric Field Imaging, PhD Thesis, MIT - Massachusetts Institute of Technology, 1999" beschrieben.

Es ist möglich, jedoch nicht zwingend, dass weitere Bestandteile des kapazitiven Sensors, z.B. eine Auswerteeinrichtung, ebenfalls im Bereich der Schuhsohle oder im Schuh angeordnet sind.

Hierbei sind mehrere Elektroden im Bereich der Schuhsohle angeordnet, wobei für jede der Elektroden ein Kapazitätswert oder ein zeitlicher Verlauf des Kapazitätswerts bestimmt wird. Der Untergrund kann dann in Abhängigkeit aller oder ausgewählter Kapazitätswerte bzw. deren zeitlicher Verläufe bestimmt werden. Insbesondere kann jeweils mindestens eine Elektrode in einem Zehenbereich, einem metatarsalen Bereich, einem Mittelfußbereich und in einem Fersenbereich der Schuhsohle angeordnet sein. Vorzugsweise ist genau eine Elektrode in einem Zehenbereich, vorzugsweise im Bereich des großen Zehs, angeordnet. Weiter sind drei Elektroden im metatarsalen Bereich angeordnet. Weiter kann genau eine Elektrode im Mittelfußbereich und genau eine Elektrode im Fersenbereich angeordnet sein.

Hierbei kann der Untergrund unmittelbar aus dem mindestens einen Kapazitätswert oder dem zeitlichen Verlauf bestimmt werden. Vorstellbar ist zum Beispiel, dass eine vorbekannte, beispielsweise vorab durch Versuche ermittelte, Zuordnung verschiedener Untergründe zu verschiedenen Kapazitätswerten oder verschiedenen zeitlichen Verläufen der Kapazitätswerte bzw. zu davon abhängigen Größen existiert. In Abhängigkeit dieser Zuordnung kann dann dem Kapazitätswert und/oder dem zeitlichen Verlauf bzw. einer davon abhängigen Größe ein Untergrund zugeordnet werden, wobei dieser Untergrund der erfindungsgemäß zu bestimmende Untergrund ist.

Insgesamt ergibt sich in vorteilhafter Weise ein einfach zu implementierendes Verfahren, welches eine zeitlich schnelle, zuverlässige und genaue Bestimmung eines Untergrunds ermöglicht.

Wie nachfolgend noch näher erläutert, kann die Information über den Untergrund genutzt werden, um beispielsweise eine Information über den Untergrund an einen Träger des Schuhs oder andere Nutzer zu übermitteln. Alternativ oder kumulativ kann die Information genutzt werden, um veränderbare Eigenschaften des Schuhs zu verändern. Dies wird nachfolgend noch näher erläutert. Auch kann, beispielsweise falls ein spannungsführender Untergrund detektiert wird, ein Warnsignal für den Nutzer oder weitere Personen in Abhängigkeit der Information erzeugt werden.

Wie nachfolgend ebenfalls noch näher erläutert, kann der Kapazitätswert und/oder der zeitliche Verlauf auch zur mittelbaren Bestimmung des Untergrundes genutzt werden, insbesondere über ein Gangbild.

Erfindungsgemäß wird in Abhängigkeit des Kapazitätswertes oder, vorzugsweise, eines zeitlichen Verlaufs eines Kapazitätswerts ein untergrundspezifisches Gangbild eines Nutzers, also eines Schuhträgers, bestimmt. Weiter wird der Untergrund, insbesondere zusätzlich, in Abhängigkeit des Gangbilds bestimmt.

Ein Gangbild im Sinne dieser Offenbarung kann insbesondere ein zu mindestens einem Zeitpunkt bestimmter Kapazitätswert der mindestens einen Elektrode sein oder umfassen. Sind mehrere Elektroden im Bereich der Schuhsohle des Schuhs angeordnet, so kann das Gangbild die Gesamtheit der für jede Elektrode oder für ausgewählte, aber nicht alle, Elektroden zu dem mindestens einen Zeitpunkt bestimmten Kapazitätswerte sein oder umfassen. Alternativ oder kumulativ kann das Gangbild auch ein Verhältnis oder mehrere Verhältnisse von Kapazitätswerten von zwei verschiedenen Elektroden umfassen, insbesondere von zwei in Fußlängsrichtung und/oder in Fußquerrichtung beabstandeten Elektroden.

Vorzugsweise ist oder umfasst das Gangbild ein zeitlicher Verlauf des Kapazitätswerts der mindestens einen Elektrode. Sind mehrere Elektroden im Bereich einer Schuhsohle des Schuhs angeordnet, so kann das Gangbild die zeitlichen Verläufe der Kapazitätswerte aller oder ausgewählter, aber nicht aller, Elektrode bezeichnen oder umfassen. Alternativ oder kumulativ kann das Gangbild auch einen zeitlichen Verlauf eines Verhältnis oder mehrerer Verhältnisse von Kapazitätswerten von zwei verschiedenen Elektroden umfassen, insbesondere von zwei in Fußlängsrichtung und/oder in Fußquerrichtung beabstandeten Elektroden.

Erfindungsgemäß umfasst das Gangbild die Kapazitätswerte oder deren zeitlichen Verläufe von Elektroden, die in Abhängigkeit einer plantare Druckverteilung oder eines zeitlichen Verlaufs der plantaren Druckverteilung erzeugt werden. Alternativ oder kumulativ kann das Gangbild mindestens ein Verhältnis von Kapazitätswerten verschiedener Elektroden oder deren zeitlichen Verläufe umfassen.

Wie vorhergehend erläutert, kann zumindest ein Anteil des Kapazitätswertes durch eine Kapazität zwischen der Elektrode und der Fußsohle des Nutzers gebildet werden oder diese Kapazität repräsentieren. Dieser Anteil verändert sich abhängig von einer Belastung des Teilbereichs des Schuhs, in dem die Elektrode angeordnet ist, durch den Fuß des Nutzers beim Laufen. Mit anderen Worten verändert sich beim Laufen durch die Gewichtsbelastung verschiedener Bereiche der Fußsohle ein Abstand zwischen der Fußsohle und der Elektrode in dem Schuhsohlenbereich, in dem die Elektrode angeordnet ist. Der Kapazitätswert ist von dem Abstand abhängig und verändert sich somit ebenfalls.

Es hat sich gezeigt, dass bei einer Abtastfrequenz von 30 Hz eine zuverlässige Bestimmung des Untergrunds möglich ist, wenn ein Zeitverlauf von Kapazitätswerten in einem Zeitintervall mit einer Länge von 0.5 Sekunden bis 4 Sekunden erfasst und ausgewertet wird.

Auch werden beim Laufen verschiedene Bereiche des Fußes belastet. Beispielsweise kann beim Abrollen zu einem bestimmten Zeitpunkt eine höhere Gewichtsbelastung in den Bereichen auftreten, in denen der Fußballen angeordnet ist, wobei zu diesem Zeitpunkt eine Belastung im Bereich der Fußferse geringer ist.

Der Kapazitätswert repräsentiert somit auch einen sich verändernden Druck und Abstand, der beim Laufen durch die sich verändernde Belastung verschiedener Schuhsohlenbereichen auftritt.

Das Gangbild, also der mindestens eine Kapazitätswert bzw. der zeitliche Verlauf von Kapazitätswerten der Elektrode oder der Elektroden, ist hierbei untergrundspezifisch, also abhängig vom Untergrund. Dies kann insbesondere bedeuten, dass sich im gleichen Abrollzustand für verschiedene Untergründe verschiedene Abstände zwischen Fußsohle und der Elektrode im Schuhsohlenbereich einstellen können. So kann z.B. der Abstand im gleichen Abrollzustand des Fußes beim Laufen auf harten Untergründen geringer als beim Laufen auf weichen Untergründen sein.

Weiter ist das Gangbild auch nutzerspezifisch. Für verschiedene Nutzer können hierbei ebenfalls verschiedene Abstände zwischen der Fußsohle und der Elektrode im gleichen Abrollzustand gegeben sein. Dies kann insbesondere der Fall sein, da verschiedene Nutzer ein verschiedenes Körpergewicht, eine verschiedene Beinlänge, eine verschiedene Fußgröße, eine verschiedene Fußform, ein verschiedenes Abrollverhalten etc. aufweisen.

Somit wird auch ein Verfahren zur Identifikation eines Nutzers beschrieben. Die Identifikation eines Nutzers in Abhängigkeit des Gangbilds kann eine unabhängige Erfindung bilden. Hierbei wird in Abhängigkeit des Kapazitätswertes oder, vorzugsweise, eines zeitlichen Verlaufs eines Kapazitätswerts ein nutzerspezifisches Gangbild bestimmt, wobei in Abhängigkeit des Gangbilds dann ein Nutzer identifiziert wird, wobei die Elektrode im Bereich einer Schuhsohle eines Schuhs angeordnet ist.

Sind mehrere Elektroden im Bereich der Schuhsohle angeordnet, so kann selbstverständlich in Abhängigkeit der Kapazitätswerte aller oder ausgewählter Elektroden und insbesondere deren zeitlichen Verläufe ein untergrundspezifisches Gangbild bestimmt werden, wobei dann in Abhängigkeit des Gangbildes der Nutzer identifiziert wird.

Hierbei kann eine Zuordnung von Gangbildern zu verschiedenen Untergründen existieren. Diese Zuordnung kann nutzerspezifisch sein. Weiter kann diese Zuordnung zum Beispiel durch Versuche ermittelt werden. In Abhängigkeit dieser Zuordnung kann dann ein Untergrund bestimmt werden, da das Gangbild oder, wie nachfolgend noch näher erläutert, Merkmale des Gangbilds, insbesondere für einen bestimmten Nutzer, einem bestimmten Untergrund zugeordnet werden kann. Weiter kann auch eine Zuordnung eines Gangbilds oder von Merkmalen des Gangbilds zu einem Nutzer existieren. Diese Zuordnung kann untergrundspezifisch sein.

Somit ist es auch möglich, dass ein Gangbild bestimmt wird, wobei in Abhängigkeit des Gangbilds ein Nutzer identifiziert wird, insbesondere in Abhängigkeit einer Zuordnung von verschiedenen Gangbildern zu verschiedenen Nutzern. Nach der Identifizierung des Nutzers kann dann eine nutzerspezifische Zuordnung von verschiedenen nutzerspezifischen Gangbildern zu verschiedenen Untergründen bestimmt werden. Diese kann dann wiederum genutzt werden, um in Abhängigkeit des Gangbilds den Untergrund zu bestimmen.

Die Bestimmung eines Untergrunds und die Bestimmung des Gangbilds in Abhängigkeit mindestens eines Kapazitätswerts oder in Abhängigkeit dessen zeitlichen Verlaufs und die Bestimmung des Untergrunds und/oder die Identifikation des Nutzers in Abhängigkeit des Gangbilds kann erfolgen, indem Merkmale des/der Kapazitätswerte bzw. der zeitlichen Verläufe oder des Gangbilds im Ortsraum und/oder im Frequenzraum bestimmt werden.

Beispielhafte Merkmale des/der Kapazitätswerte bzw. der zeitlichen Verläufe können z.B. sein: eine maximale Amplitude, eine signifikante Frequenz, ein Spektral Flux, eine spektrale Energie, ein Wert einer logarithmischen Wahrscheinlichkeitsfunktion, eine normierte Differenz zwischen den Kapazitätswerten mehrerer Sensoren, eine durchschnittliche absolute Differenz zwischen den Kapazitätswerten mehrerer Sensoren, eine Kreuzung eines Mittelwertes, ein geometrischer Mittelwert, die Summe eines einzelnes Quartils, die Differenz zwischen dem dritten und dem ersten Quartil (interquartile Reichweite), der maximale Kapazitätswert in einem Zeitfenster vorbestimmter Größe, die Differenz zwischen dem Minimum und Maximum, die statistische Schiefe (Skewness), sowie die statistische Breite (Kurtosis).

Verschiedene Merkmale oder verschiedene Merkmalkombination können hierbei verschiedenen Untergründen zugeordnet sein. Auch kann einem Nutzer ein bestimmtes Merkmal oder eine bestimmte Merkmalkombination zugeordnet sein. Untergrundspezifische bzw. nutzerspezifische Merkmale oder Merkmalskombinationen können hierbei in Testverfahren oder Trainingsverfahren bestimmt werden.

In Abhängigkeit der Merkmale kann dann ein Klassifikationsverfahren durchgeführt werden, z.B. ein mittels eines sogenannten Bayes-Net-Klassifikator, eines Naive-Bayes-Klassifikator, eines Random-Forest-Klassifikator, eines Nearest Neighbor-Klassifikator und oder eines Sequential-Minimal-Optimization-Klassifikator. Mittels des Klassifikationsverfahrens ist dann ein Untergrund, ein Gangbild oder ein Nutzer bestimmbar bzw. identifizierbar.

Die Bestimmung des Untergrunds kann hierbei durch Verfahren des sogenannten maschinellen Lernens erfolgen. Auch die Bestimmung des Nutzers kann durch Verfahren des maschinellen Lernens bestimmt werden.

Das Gangbild kann auch zu Therapie- oder Diagnosezwecken verwendet, insbesondere ausgewertet, werden. Hierzu kann das Gangbild an ein übergeordnetes System übertragen werden.

Das Gangbild kann weiter einen biometrischen Datensatz, insbesondere einen nutzerspezifischen biometrischen Datensatz, bilden. Alternativ kann in Abhängigkeit des Gangbilds ein biometrisches Merkmal erzeugt werden.

Das Gangbild oder der biometrische Datensatz oder das biometrische Merkmal kann dann zu Identifizierungs- oder Autorisierungszwecken genutzt werden. So kann ein Nutzer in Abhängigkeit des Gangbilds biometrisch identifiziert werden.

Beispielsweise kann das Gangbild, der biometrische Datensatz oder das biometrische Merkmal mittels einer Kommunikations- oder Datenübertragungseinrichtung an ein externes System, beispielsweise ein portables Endgerät, übertragen werden. In dem externen System kann dann das biometrische Merkmal zu Identifizierungszwecken und/oder Autorisierungszwecken genutzt werden. Beispielsweise kann ein Zugang zu dem System oder Funktionen des Systems nur dann erlaubt werden, wenn das Gangbild einem vorbestimmten Gangbild entspricht oder der biometrische Datensatz einem vorbestimmten nutzerspezifischen Datensatz entspricht oder das biometrische Merkmal einem nutzerspezifischen Merkmal entspricht.

Hierdurch ergibt sich in vorteilhafter Weise eine weitere, alternative Möglichkeit zur Bestimmung eines Untergrunds, die ebenfalls eine zuverlässige und genaue Bestimmung ermöglicht. Auch ergibt sich die Möglichkeit einer redundanten Bestimmung des Untergrundes, wobei die Zuverlässigkeit in diesem Fall erhöht wird.

In einer weiteren Ausführungsform wird ein Grundkopplungsanteil des mindestens einen Kapazitätswertes bestimmt. Weiter wird der Untergrund, insbesondere zusätzlich, in Abhängigkeit des Grundkopplungsanteils, insbesondere einer Höhe oder eines Betrags des Grundkopplungsanteils bestimmt.

Der erfindungsgemäß bestimmte Kapazitätswert kann sich hierbei aus mindestens zwei Anteilen zusammensetzen. Ein erster Anteil ist der Grundkopplungsanteil. Dieser repräsentiert eine Kapazität, die zwischen dem zu bestimmenden Untergrund und der Elektrode vorhanden ist. Mindestens ein weiterer Anteil des Kapazitätswertes kann aus einer Kapazität bestehen, die zwischen der Elektrode und einer Fußsohle des Schuhträgers vorhanden ist. Versuche haben gezeigt, dass der Grundkopplungsanteil charakteristisch für einen Untergrund ist. Insbesondere kann der Grundkopplungsanteil für verschiedene Untergründe unterschiedlich sein. Somit kann eine Zuordnung von Grundkopplungsanteilen zu bestimmten Untergründen existieren, die beispielsweise vorab durch Versuche ermittelt wird. In Abhängigkeit dieser Zuordnung kann dann einem ermittelten Grundkopplungsanteil ein Untergrund zugeordnet werden.

Der Grundkopplungsanteil kann hierbei mittels dem Fachmann bekannten Verfahren, z.B. Filterverfahren, aus dem mindestens einen Kapazitätswert oder dem mindestens einen zeitlichen Verlauf oder aber aus Kapazitätswerten mehrerer Elektroden bzw. den zeitlichen Verläufen von Kapazitätswerten mehrerer Elektroden bestimmt werden. Ein beispielhaftes Verfahren zur Bestimmung des Grundkopplungsanteils wird nachfolgend noch näher erläutert. Hierbei ist es rein theoretisch möglich, den Grundkopplungsanteil in Abhängigkeit eines einzigen Kapazitätswerts unmittelbar, z.B. einem zu einem Zeitpunkt bestimmten Kapazitätswerts, zu bestimmen. Aufgrund von Sensorrauschen und gegebenenfalls auftretenden Messfehlern wird der Grundkopplungsanteil jedoch bevorzugt in Abhängigkeit von mehreren Kapazitätswerten eines zeitlichen Verlaufs von Kapazitätswerten bestimmt.

Für einen zeitlichen Verlauf wird eine Abtastfrequenz zur Bestimmung von Kapazitätswerten eingestellt, die z.B. 30 Hz betragen kann. In diesem Fall kann der Grundkopplungsanteil z.B. aus den Kapazitätswerten bestimmt werden, die innerhalb eines Zeitintervalls z.B. einer halben Sekunde oder länger erzeugt wurden.

Vorzugsweise wird also der Untergrund in Abhängigkeit des Grundkopplungsanteils und des Gangbilds bestimmt. Hierbei ist der Grundkopplungsanteil abhängig von einer elektrischen Isolation des Untergrunds, während das Gangbild abhängig von physikalischen Eigenschaften, insbesondere mechanischen Eigenschaften, des Untergrunds ist.

Hierzu kann insbesondere eine Zuordnung einer Kombination aus Gangbild (oder Merkmalen des Gangbilds) und Grundkopplungsanteil zu einem Untergrund existieren, wobei die Zuordnung in Testverfahren ermittelt und dann zur Bestimmung des Untergrunds genutzt werden kann.

Theoretisch ist es möglich, den Grundkopplungsanteil und den mindestens einen weiteren Anteil des Kapazitätswertes, der durch eine Kapazität zwischen der Elektrode und der Fußsohle des Nutzers gebildet wird, unabhängig voneinander zu erfassen. Hierzu kann es erforderlich sein, eine Anordnung von mindestens drei Elektroden zu verwenden. Hierbei kann eine erste Elektrode derart angeordnet und/oder ausgebildet sein, dass mittels der ersten Elektrode der Grundkopplungsanteil erfassbar ist. Eine zweite Elektrode kann derart angeordnet und/oder ausgebildet sein, dass mittels der zweiten Elektrode der mindestens eine weitere Anteil erfassbar ist. Eine dritte Elektrode kann als Schirmungselektrode zwischen der ersten und der zweiten Elektrode angeordnet sein.

Somit können das Gangbild in Abhängigkeit der Kapazitätswerte der zweiten Elektrode und der Grundkopplungsanteil in Abhängigkeit der Kapazitätswerte der ersten Elektrode bestimmt werden.

Hierdurch ergibt sich in vorteilhafter Weise eine rechentechnisch einfache, sowie sichere und schnell durchzuführende Bestimmung des Untergrunds.

Weiter erfindungsgemäß wird ein zeitlicher Verlauf des Kapazitätswerts bestimmt, wobei ein Gleichanteil oder ein Mittelwert eines Zeitfensters des zeitlichen Verlaufs bestimmt wird, wobei der Grundkopplungsanteil in Abhängigkeit des Gleichanteils oder des Mittelwerts bestimmt wird. Das Zeitfenster kann hierbei eine vorbestimmte Zeitdauer aufweisen. Insbesondere kann der Grundkopplungsanteil der Gleichanteil oder der Mittelwert sein.

Wie nachfolgend noch näher erläutert, kann ein Kapazitätswert der Elektrode sich aufgrund einer sich verändernden Belastung des Schuhbereichs, in dem die Elektrode angeordnet ist, beim Laufen verändern. In diesem Fall wird auch ein sich zeitlich verändernder Kapazitätswert bestimmt. Der zeitliche Verlauf kann hierbei ein quasi periodischer Verlauf mit einem Gleichanteil sein Bewegt sich der Fußgänger beispielsweise von Asphalt auf Teppich, so kann sich der Gleichanteil und/oder der Mittelwert der Kapazitätswerte verändern, insbesondere erhöhen oder verringern.

Somit wird in vorteilhafter Weise eine rechentechnisch einfache und schnelle Bestimmung des Grundkopplungsanteils und somit des Untergrunds ermöglicht.

In einer weiteren Ausführungsform wird ein spannungsführender Untergrund und/oder ein feuchter Untergrund in Abhängigkeit des Kapazitätswerts oder eines zeitlichen Verlaufs des Kapazitätswerts identifiziert. Die Identifizierung kann hierbei wiederum, insbesondere entsprechend den vorhergehenden Erläuterungen, durch Verfahren des maschinellen Lernens erfolgen. Spannungsführende Untergründe können sich insbesondere durch ein Signal-zu-Rausch-Verhältnis, eine Signalbereichsweite und/oder einen Signalversatz von nicht spannungsführenden Untergründen unterscheiden. Somit kann ein spannungsführender Untergrund insbesondere in Abhängigkeit mindestens einer dieser Merkmale identifiziert werden. Feuchte Untergründe können sich insbesondere durch eine breitere Signalbereichsweite von trockenen Untergründen unterscheiden. Somit kann ein feuchter Untergrund insbesondere in Abhängigkeit der Signalbereichsweite identifiziert werden. Hierbei bezeichnet ein Signal einen Kapazitätswert.

Ein spannungsführender Untergrund kann insbesondere einen Untergrund bezeichnen, dessen Potential von einem Massepotential, insbesondere einem neutralen Massepotential, verschieden ist. Wird ein spannungsführender und/oder feuchter Untergrund identifiziert, so kann ein Warnsignal für einen Nutzer oder ein übergeordnetes System, beispielsweise ein Gebäude-Sicherheitssystem, erzeugt werden, z.B. von der Auswerteeinrichtung. Die Identifizierung eines spannungsführenden Untergrunds kann beispielsweise auch von Interesse für die Einhaltung einer Betriebssicherheit, z.B. in Fabriken, sein.

Wird ein feuchter Untergrund identifiziert, so kann insbesondere mindestens eine Eigenschaft des Schuhes derart eingestellt werden, dass ein Halt des Schuhs auf dem feuchten Untergrund mit veränderten Eigenschaften des Schuhs im Vergleich zu dem Halt auf dem nassen Untergrund mit den unveränderten, ursprünglichen Eigenschaften verbessert wird. Selbstverständlich kann alternativ oder kumulativ ein Warnsignal für den Nutzer oder ein übergeordnetes System erzeugt werden.

In einer bevorzugten Ausführungsform sind mehrere Elektroden im Bereich der Schuhsohle angeordnet, wobei Kapazitätswerte oder zeitliche Änderungen der Kapazitätswerte ausgewählter oder aller Elektroden bestimmt werden, wobei das untergrundspezifische Gangbild in Abhängigkeit dieser Kapazitätswerte oder dieser zeitlichen Änderungen bestimmt wird. Durch mehrere Elektroden ergibt sich in vorteilhafter Weise eine höhere räumliche Auflösung von Belastungszuständen oder eines zeitlichen Verlaufes der Belastungszustände und somit eine bessere und genauere Identifikation eines Gangbildes. Insbesondere kann das Gangbild auch in Abhängigkeit der relativen Unterschiede zwischen den verschiedenen Kapazitätswerten bzw. deren zeitlichen Verläufe bestimmt werden.

Hierdurch wird in vorteilhafter Weise eine Zuverlässigkeit und Genauigkeit der Bestimmung erhöht.

In einer weiteren Ausführungsform ist ein untergrundspezifisches Gangbild ein angelerntes Gangbild. Dies kann bedeuten, dass ein Nutzer auf verschiedenen Untergründen läuft, wobei die beim Laufen erfassten Kapazitätswerte bzw. deren zeitliche Verläufe abgespeichert werden. Diesen Kapazitätswerten bzw. zeitlichen Verläufen kann dann der Untergrund, auf dem der Nutzer läuft, zugeordnet werden. Dies kann beispielsweise durch die zuvor erläuterten oder weitere geeignete Klassifikations-/ Zuordnungsverfahren erfolgen.

Hierdurch ergibt sich in vorteilhafter Weise eine hohe Zuverlässigkeit bei der Bestimmung verschiedener Untergründe.

In einer weiteren Ausführungsform ist die mindestens eine Elektrode eine flächige Elektrode. Hierdurch ergibt sich in vorteilhafter Weise die bessere Erkennung der kapazitiven Grundkopplung.

In einer weiteren Ausführungsform wird in Abhängigkeit des erfindungsgemäß bestimmten Untergrunds mindestens eine Eigenschaft des Schuhs eingestellt. Beispielsweise kann eine Elastizität der Sohle des Schuhs eingestellt werden. Hierzu kann der Schuh beispielsweise eine Sohle mit aktiv veränderbaren Elastizitätseigenschaften umfassen. Somit dient der erfindungsgemäß bestimmte Untergrund zur Steuerung einer Eigenschaft des Schuhs. Somit wird auch ein Verfahren zur Einstellung mindestens einer Eigenschaft eines Schuhs beschrieben, wobei ein Untergrund gemäß einer der in dieser Offenbarung beschriebenen Ausführungsformen bestimmt und die mindestens eine Eigenschaft des Schuhs in Abhängigkeit des Untergrunds eingestellt wird. Beispielsweise kann für harte Untergründe eine höhere Elastizität als für weichere Untergründe eingestellt werden.

Alternativ oder kumulativ kann in Abhängigkeit des bestimmten Untergrunds ein Warnsignal erzeugt werden. Beispielsweise kann ein Warnsignal erzeugt werden, wenn ein spannungsführender Untergrund bestimmt wird. Hierzu kann der Schuh eine Einrichtung zur Erzeugung des Warnsignals umfassen, beispielsweise eine Lautsprechereinrichtung.

Somit wird auch ein Verfahren zur Erzeugung eines Warnsignals beschrieben, wobei ein Untergrund gemäß einer der in dieser Offenbarung beschriebenen Ausführungsformen bestimmt wird, wobei in Abhängigkeit des bestimmten Untergrunds ein Warnsignal erzeugt wird.

Selbstverständlich können Informationen bezüglich des bestimmten Untergrunds oder die bestimmten Kapazitätswerte auch zu einer externen Einrichtung, beispielsweise einer externen Servereinrichtung, übertragen werden. Hierbei kann die Bestimmung des Untergrunds in externen Einrichtungen erfolgen. Weiter kann durch die externe Einrichtung oder eine damit daten- und/oder signaltechnisch verbundene Einrichtung eine Lokalisierung oder eine Navigationsanwendung für den Nutzer durchgeführt werden. So ist es z.B. vorstellbar, dass eine Information über den bestimmten Untergrund an ein Endgerät des Nutzers, beispielsweise ein Mobilfunktelefon oder ein Tablet - PC, übertragen wird. Auch können Informationen zur Position des Nutzers in Abhängigkeit des bestimmten Untergrunds bestimmt und dann an ein Endgerät des Nutzers übertragen werden. Auch können Navigationsinformationen an das Endgerät übertragen werden.

Diese Informationen können beispielsweise von der vorhergehend erläuterten externen Einrichtung oder einer im Schuh angeordneten Kommunikationseinrichtung übertragen werden.

In einer weiteren Ausführungsform wird eine Haltung eines Nutzers in Abhängigkeit des Kapazitätswerts oder eines zeitlichen Verlaufs des Kapazitätswerts bestimmt. Auch die Haltung kann hierbei durch Verfahren des maschinellen Lernens bestimmt werden. Die Haltung kann eine Fuß- oder Körperhaltung sein. So kann z.B. eine sitzende Haltung, eine stehende Haltung, eine liegende Haltung des Nutzers bestimmt werden.

In Abhängigkeit einer derart bestimmten Haltung kann ein Warnsignal für den Nutzer erzeugt werden. Insbesondere kann ein Warnsignal erzeugt werden, wenn in Abhängigkeit der bestimmten Haltung ein Fallrisiko des Nutzers bestimmt wird, welches größer als ein vorbestimmtes Risiko ist.

Weiter kann die Bestimmung der Haltung des Nutzers genutzt werden, um eine Patientenüberwachung durchzuführen, beispielsweise eine Aktivitätsüberwachung. Auch kann in Abhängigkeit der derart bestimmten Haltung ein, insbesondere zeitliches, Aktivitätsprofil des Nutzers bestimmt werden, welches z.B. genutzt werden kann, um eine verbesserte Diagnose und/oder Therapie für den Nutzer zu ermöglichen.

Sind mehr als eine, insbesondere mehr als zwei, Elektrode(n) im Bereich der Schuhsohle des Schuhs angeordnet, so kann die Haltung in Abhängigkeit ausgewählter, aber nicht aller, vorzugsweise in Abhängigkeit der Kapazitätswerte von genau zwei Elektroden bestimmt werden. Insbesondere kann die Haltung in Abhängigkeit der Kapazitätswerte einer Elektrode, die im Mittenbereich des Fußes, und einer Elektrode, die im Absatzbereich des Fußes angeordnet ist, bestimmt werden.

Die Bestimmung einer Haltung des Nutzers in Abhängigkeit des Kapazitätswerts oder eines zeitlichen Verlaufs des Kapazitätswerts kann eine unabhängige Erfindung bilden. Somit wird auch ein Verfahren zur Bestimmung einer Haltung eines Nutzers beschrieben. Hierbei wird in Abhängigkeit des Kapazitätswertes der mindestens einen Elektrode oder, vorzugsweise, eines zeitlichen Verlaufs eines Kapazitätswerts der mindestens einen Elektrode eine Haltung des Nutzers bestimmt, wobei die Elektrode im Bereich einer Schuhsohle eines Schuhs angeordnet ist.

Weiter vorgeschlagen wird eine Vorrichtung zur Bestimmung eines Untergrunds, wobei die Vorrichtung mindestens einen Trägerkörper, mindestens eine Elektrode und mindestens eine Auswerteeinrichtung umfasst. Zumindest ein Teil der Vorrichtung, insbesondere der Trägerkörper, ist im Bereich einer Schuhsohle eines Schuhs anordenbar. Dies kann bedeuten, dass der Teil der Vorrichtung als Teil einer Schuhsohle ausgebildet, insbesondere als eine Einlegesohle. Auch kann dies bedeuten, dass der Trägerkörper zwischen der Fußsohle eines Nutzers und einer Unterseite, also einer Lauffläche, des Schuhs angeordnet ist. Die Elektrode ist in, auf oder an dem Trägerkörper angeordnet. Ein möglicher Trägerkörper kann insbesondere aus Plexiglas oder einem weiteren flexiblen elektrisch nicht leitenden Material, insbesondere aus Kunststoff, bestehen. Die Elektrode kann beispielsweise aus Kupfer bestehen, insbesondere aus Kupferband ausgeschnitten sein oder als aus einem leitenden Polymer bzw. leitfähigem Faden bestehen.

Weiter ist mindestens ein Kapazitätswert der mindestens einen Elektrode bestimmbar, insbesondere durch die Auswerteeinrichtung. Die Auswerteeinrichtung kann hierbei im Schuh, beispielsweise ebenfalls im Bereich der Schuhsohle, oder außerhalb des Schuhs angeordnet sein. Insbesondere in diesem Fall, jedoch nicht ausschließlich in diesem Fall, kann die Vorrichtung auch eine Signalübertragungseinrichtung umfassen, wobei mittels der Signalübertragungseinrichtung Ausgangssignale der Elektrode oder Informationen, die von der Auswerteeinrichtung bestimmt wurden, an eine externe Einrichtung übertragen werden können. Z.B. können Ausgangssignale der Elektrode an eine externe Auswerteeinrichtung übertragen werden, die dann den Kapazitätswert bestimmen kann.

Weiter ist, insbesondere durch die Auswerteeinrichtung der Untergrund in Abhängigkeit des Kapazitätswerts oder eines zeitlichen Verlaufs des Kapazitätswerts bestimmbar oder identifizierbar. Dies und entsprechende Vorteile wurden vorgehend erläutert.

Insbesondere ist in Abhängigkeit des Kapazitätswertes oder, vorzugsweise, eines zeitlichen Verlaufs eines Kapazitätswerts ein untergrundspezifisches Gangbild eines Nutzers, also eines Schuhträgers, bestimmbar. Weiter ist der Untergrund in Abhängigkeit des Gangbilds bestimmbar.

Weiter ist es möglich, dass ein Nutzer in Abhängigkeit des Gangbilds identifizierbar ist. Weiter ist es möglich, dass eine Haltung in Abhängigkeit des Kapazitätswertes oder, vorzugsweise, eines zeitlichen Verlaufs eines Kapazitätswerts bestimmbar ist. Weiter ist es möglich, dass ein feuchter Untergrund und/oder ein spannungsführender Untergrund in Abhängigkeit des Kapazitätswertes oder, vorzugsweise, eines zeitlichen Verlaufs eines Kapazitätswerts bestimmbar ist.

Die Vorrichtung ermöglicht in vorteilhafter Weise die Durchführung eines Verfahrens gemäß einer in dieser Offenbarung beschriebenen Ausführungsformen. Somit ist die Vorrichtung entsprechend ausgebildet.

An einer Oberseite des Trägerkörpers kann eine Isolationsschicht angeordnet sein, die die Elektroden gegenüber einer Fußsohle elektrisch isoliert. Weiter kann an einer Oberseite des Trägerköpers, insbesondere über der Isolationsschicht, eine feuchtigkeitsableitende Schicht angeordnet sein. An einer Unterseite des Trägerkörpers kann ebenfalls eine Isolationsschicht angeordnet sein. Alternativ oder kumulativ kann an der Unterseite eine Haftschicht, insbesondere eine Silikonschicht, angeordnet sein, um eine gute Haftung zwischen dem Trägerkörper und dem Schuh zu gewährleisten.

Weiter kann die Vorrichtung mindestens eine Energiespeichereinrichtung und/oder Verbindungselemente zur elektrischen und/oder signaltechnischen Verbindung der verschiedenen Bestandteile der Vorrichtung umfassen. Diese können im Schuh, insbesondere im Bereich der Schuhsohle, beispielsweise ebenfalls am Trägerkörper, angeordnet sein.

In einer weiteren Ausführungsform ist zumindest ein Teil der Vorrichtung als Einlegesohle ausgebildet. Hierdurch ergibt sich in vorteilhafter Weise, dass die Vorrichtung einfach in existierende Schuhe integriert werden kann.

In einer weiteren Ausführungsform umfasst die Vorrichtung mindestens eine Isolationsschicht bzw. Pufferschicht, wobei die Isolationsschicht bzw. Pufferschicht im eingelegten Zustand des Trägerkörpers mit der Elektrode über der mindestens einen Elektrode angeordnet ist. Im eingelegten Zustand kann die Elektrode zwischen Fußsohle und Unterseite des Schuhs angeordnet sein. Die Isolationsschicht bzw. Pufferschicht kann insbesondere aus Schaumstoff bestehen.

Das vorgeschlagene Verfahren und die vorgeschlagene Vorrichtung kann auch dazu genutzt werden, unerwünschte oder ungesunde Belastungszustände eines Nutzers zu detektieren. So kann z.B. detektiert werden, ob ein Nutzer zu schwere Sachen trägt oder hebt. Dies kann beispielsweise durch den plantaren Druck im Gangbild bestimmt werden, welches sich in Abhängigkeit der Kapazitätswerte bzw. deren zeitlicher Verläufe ergibt.

Das vorgeschlagene Verfahren und die vorgeschlagene Vorrichtung kann auch dazu genutzt werden, eine bestimmte Körperhaltung eines Nutzers zu detektieren. So kann z.B. detektiert werden, ob ein Nutzer aufrecht steht, sich bückt oder sitzt. Dies wird beispielsweise durch das plantare Druckbild deutlich, welches sich in Abhängigkeit der Kapazitätswerte bzw. deren zeitlicher Verläufe ergibt.

Die Erfindung wird anhand eines Ausführungsbeispiels näher erläutert. Die Figuren zeigen:

Fig. 1    eine schematische Darstellung von Kapazitäten

Fig. 2    eine schematische Darstellung eines Schuhs,

Fig. 3    eine schematische Darstellung einer erfindungsgemäßen Vorrichtung und

Fig. 4    eine schematische Darstellung einer erfindungsgemäßen Vorrichtung in einer weiteren Ausführungsform.

Nachfolgend bezeichnen gleiche Bezugszeichen Elemente mit gleichen oder ähnlichen technischen Merkmalen.

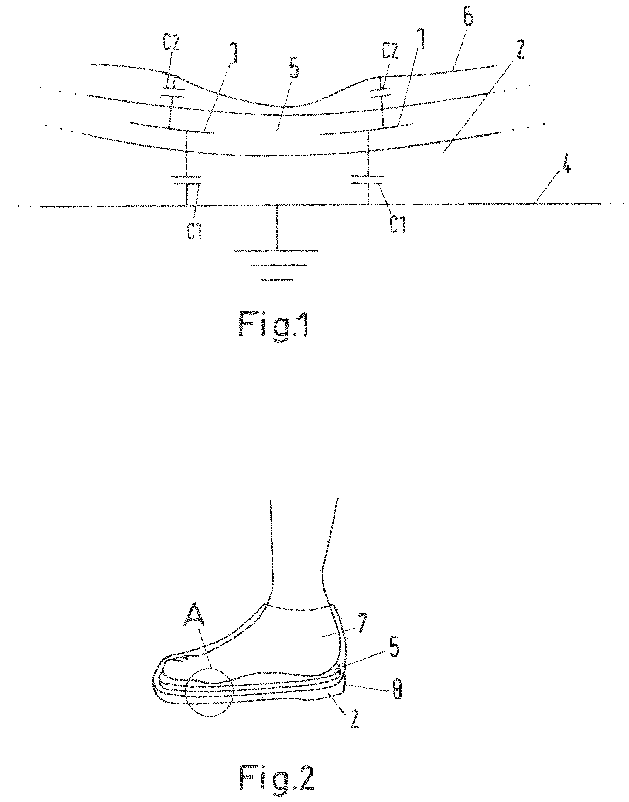

In Fig. 1 zeigt eine schematische Darstellung von Kapazitätswerten von Elektroden 1, die im Bereich einer Schuhsohle 2 eines Schuhs 8 (siehe Fig. 2) angeordnet sind. Hierbei ist dargestellt, dass die Schuhsohle 2 auf eine Oberfläche eines Untergrunds 4 aufgesetzt ist. Die Elektroden 1 sind hierbei in einem Trägerkörper 5 angeordnet. Der Trägerkörper 5 kann beispielsweise aus flexiblem Plexiglas bestehen.

Ein Kapazitätswert einer Elektrode 1 setzt sich zumindest aus zwei Anteilen zusammen. Ein erster Kapazitätsanteil C1 kann auch als Grundkopplungsanteil bezeichnet werden und repräsentiert eine Kapazität zwischen der Elektrode 1 und dem Untergrund 4. Ein weiterer Kapazitätsanteil C2 repräsentiert eine Kapazität zwischen der Elektrode 1 und einer Fußsohle 6 eines Nutzers (Schuhträgers).

Die Kapazität zwischen der Elektrode 1 und der Fußsohle 6 bzw. dem Untergrund 4 kann z.B. gemäß

$$C = e_r \times e_0 \times (A/d) \qquad\qquad \text{(Formel 1)}$$

bestimmt werden, wobei $e_r$ eine relative statische Dielektrizitätskonstante, $e_0$ eine elektrische Feldkonstante, A eine Fläche der Elektrode 1 und d den Abstand zwischen der Elektrode 1 und dem Untergrund 4 bzw. der Fußsohle 6 bezeichnet. Die Elektrode 1 kann ebenfalls flexibel sein oder aus flexiblem Material bestehen. Formel 1 beschreibt die Kapazität eines perfekten Plattenkondensators. Somit kann Formel 1 genutzt werden, um eine theoretische Kapazität zu bestimmen, z.B. zwischen der Elektrode 1 und dem Untergrund 4 mit einem Abstand d. Diese Kapazität kann dann dem Grundkopplungsanteil entsprechen. Es ist allerdings zu beachten, dass eine tatsächliche gemessene Kapazität von dieser theoretischen Kapazität abweichen kann.

In einem Verfahren zur Bestimmung des Untergrunds 4, insbesondere einer Art des Untergrunds oder einer Eigenschaft des Untergrunds 4, werden die Kapazitätswerte der Elektroden 1 bestimmt, wobei sich die Kapazitätswerte zumindest, nicht zwingendermaßen aber ausschließlich, aus den erläuterten Anteilen zusammensetzen.

Das Bestimmen des Kapazitätswerts einer Elektrode 1 erfolgt, indem die Elektrode 1 zyklisch geladen und entladen wird. Die zeitliche Dauer des Ladevorgangs, beispielsweise von einem entladenen Zustand bis zu einem vollständig geladenen Zustand, ermöglicht die Bestimmung des Kapazitätswerts.

Fig. 2 zeigt eine schematische Darstellung eines Fußes 7, der in einem Schuh 8 angeordnet ist. Weiter dargestellt ist der Trägerkörper 5 sowie eine Schuhsohle 2. Der mit dem Kreis A markierte Ausschnitt kann beispielsweise die in Fig. 1 dargestellten Elektroden 1 umfassen.

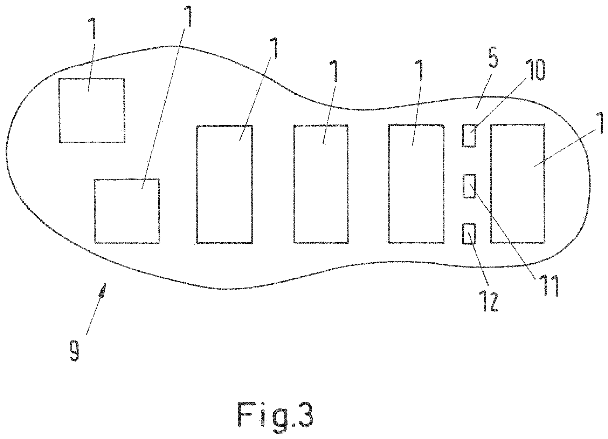

Fig. 3 zeigt eine exemplarische Darstellung einer erfindungsgemäßen Vorrichtung 9. Die erfindungsgemäße Vorrichtung 9 zur Bestimmung eines Untergrunds 4 umfasst einen Trägerkörper 5, der im dargestellten Ausführungsbeispiel als Einlegesohle ausgebildet sein kann. Weiter umfasst die Vorrichtung sechs Elektroden 1, die als Flächenelektroden ausgebildet sind. Weiter umfasst die Vorrichtung eine Auswerteeinrichtung 10 sowie eine Energiespeichereinrichtung 11 und eine Signalübertragungseinrichtung 12. Die Elektroden 1 können hierbei wie die Auswerteeinrichtung 10, die Energiespeichereinrichtung 11 und die Signalübertragungseinrichtung 12 in dem Trägerkörper 5 angeordnet sein.

Nicht dargestellt sind elektrische und signaltechnische Verbindungen zwischen den Elektroden 1, der Auswerteeinrichtung 5, der Energiespeichereinrichtung 11 und der Signalübertragungseinrichtung 12. Auch diese Verbindungen können in dem Trägerkörper 5 angeordnet sein.

Mittels der Auswerteeinrichtung 5 können die Kapazitätswerte der Elektroden 1 bestimmt werden. Weiter kann mittels der Auswerteeinrichtung 5 in Abhängigkeit der Kapazitätswerte oder eines zeitlichen Verlaufs der Kapazitätswerte ein Untergrund bestimmt, d.h. identifiziert, werden.

Hierzu ist es möglich, dass die Kapazitätswerte, insbesondere jedoch zeitliche Verläufe der Kapazitätswerte, bestimmt werden.

Insbesondere kann ein Grundkopplungsanteil dieser Kapazitätswerte aus den jeweiligen zeitlichen Verläufen bestimmt werden. Weiter kann der Untergrund in Abhängigkeit des Grundkopplungsanteils, insbesondere in Abhängigkeit einer vorbekannten Zuordnung von Grundkopplungsanteilen zu verschiedenen Untergründen, bestimmt werden.

Alternativ oder kumulativ kann in Abhängigkeit der zeitlichen Verläufe der Kapazitätswerte ein untergrundspezifisches Gangbild eines Schuhträgers bestimmt werden, wobei der Untergrund 4 dann (zusätzlich) in Abhängigkeit des Gangbilds bestimmt wird. Beim Abrollen des Fußes beim Laufen belastet der Fuß 7 (siehe Fig. 2) die in Fig. 3 dargestellte Einlegesohle in verschiedenen Bereichen verschieden stark. Bei hoher Belastung ist ein Abstand zwischen der Fußsohle 6 (siehe Fig. 1) und der im belastenden Bereich angeordneten Elektrode 1 kleiner als bei einer niedrigeren Belastung. Diese sich verändernden Abstände bedingen sich verändernde Kapazitätswerte. Ein Gangbild kann somit durch bestimmte zeitliche Verläufe charakterisiert sein.

Weiter kann einem Gangbild ein bestimmter Untergrund zugeordnet sein. In Abhängigkeit des/der zeitlichen Verlaufs/Verläufe kann ein Gangbild identifiziert werden, wobei in Abhängigkeit des Gangbilds dann ein Untergrund identifiziert wird.

Die Auswerteeinrichtung 5 kann mittels der Signalübertragungseinrichtung 12 Informationen zu dem identifizierten Untergrund oder die mittels der Auswerteeinrichtung 5 bestimmten Kapazitätswerte an eine externe Einrichtung, beispielsweise eine externe Servereinrichtung oder ein mobiles Endgerät des Nutzers, oder an ein übergeordnetes System übertragen.

Weiter ist möglich, dass die Auswerteeinrichtung 5 signal- und/oder datentechnisch mit einer Steuereinrichtung zur Steuerung einer Elastizität des Schuhs 8, insbesondere der Schuhsohle 2, verbunden ist. Diese Steuereinrichtung (nicht dargestellt) kann dann in Abhängigkeit der bestimmten Kapazitätswerte oder des bestimmten Untergrundes eine Elastizität des Schuhs 8 einstellen.

Insbesondere kann mittels der Auswerteeinrichtung 5 in Abhängigkeit der Kapazitätswerte oder eines zeitlichen Verlaufs der Kapazitätswerte ein spannungsführender Untergrund und/oder ein feuchter Untergrund identifiziert werden.

Auch kann mittels der Auswerteeinrichtung 5 in Abhängigkeit der Kapazitätswerte oder eines zeitlichen Verlaufs der Kapazitätswerte eine Haltung des Nutzers bestimmt werden. Auch kann mittels der Auswerteeinrichtung 5 in Abhängigkeit des Gangbilds ein Nutzer identifiziert werde werden.

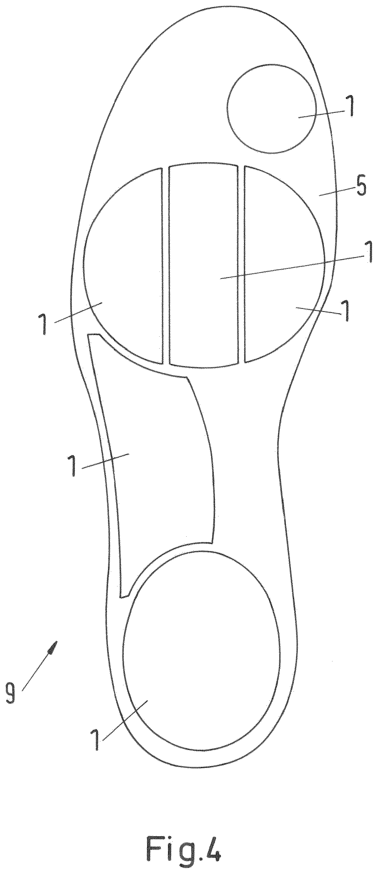

Fig. 4 zeigt eine exemplarische Darstellung einer erfindungsgemäßen Vorrichtung 9 in einer weiteren Ausführungsform. Wie bei dem in Fig. 3 dargestellten Ausführungsbeispiel umfasst die Vorrichtung 9 zur Bestimmung eines Untergrunds 4 einen Trägerkörper 5, der als Einlegesohle ausgebildet ist. Weiter umfasst die Vorrichtung 9 sechs Elektroden 1, die als Flächenelektroden ausgebildet sind. Im Unterschied zu der in Fig. 3 dargestellten Ausführungsform weisen diese Elektroden 1 verschiedene Geometrien auf, insbesondere eine ovale Geometrie, eine vollkreisförmige Geometrie, eine halbkreisförmige Geometrie und eine rechteckige Geometrie sowie eine Freiform-Geometrie. Die Elektroden 1, insbesondere deren Geometrien, sind hierbei derart angeordnet und/oder ausgebildet, dass diese in Bereichen der Einlegesohle angeordnet sind, die beim Laufen durch einen Fuß besonders stark belastet werden. So ist dargestellt, dass eine Elektrode 1, insbesondere eine ovale Elektrode, im Bereich der Ferse angeordnet ist. Eine vollkreisförmige Elektrode 1 ist im Bereich des großen Zehs angeordnet. Drei Elektroden 1, insbesondere zwei halbkreisförmige und eine rechteckige Elektrode 1, sind im Bereich des Ballens / Metatarsal angeordnet. Diese Elektroden 1 sind hierbei nebeneinander entlang einer Linie angeordnet, die von der Fußaußenseite zur Fußinnenseite orientiert ist. Hierbei ist die rechteckige Elektrode 1 zwischen den halbkreisförmigen Elektroden 1 angeordnet, wobei die gekrümmten Randabschnitte der halbkreisförmigen Elektroden 1 hin zur Fußaußenseite bzw. hin zur Fußinnenseite orientiert sind. Eine Elektrode 1 in Freiform ist im Bereich des Fußaußenrandes / Mittelfußes angeordnet.

Nicht in Fig. 4 dargestellt sind eine Auswerteeinrichtung 10 sowie eine Energiespeichereinrichtung 11 und eine Signalübertragungseinrichtung 12.

Versuche haben gezeigt, dass mit dem erfindungsgemäßen Verfahren eine Identifikation eines Nutzers in Abhängigkeit eines Gangbilds mit einer Zuverlässigkeit von 70% bis 100% möglich ist. Je länger die Zeitdauer der ausgewerteten zeitlichen Verläufe, desto höher die Zuverlässigkeit der Identifikation. So konnte gezeigt werden, dass die einwandfreie Identifikation von 13 Nutzern eine Signalerfassung in einem Zeitfenster mit einer Dauer von ca. 8 Sekunden erforderte, in dem der jeweilige Nutzer normal ging. Versuche haben weiter gezeigt, dass sechs verschiedene Untergründe mit einer Zuverlässigkeit von in einem Bereich von 70% bis 90% identifizierbar sind. Diese Genauigkeitswerte wurden bei Signalerfassung in einem Zeitfenster von 4,5 Sekunden bei normalem Gehen erreicht. Auch für die Identifikation erhöht sich die Genauigkeit entsprechend mit der Vergrößerung des Zeitfensters.

Bezugszeichenliste

1   Elektrode
2   Schuhsohle
4   Untergrund
5   Trägerkörper
6   Fußsohle
C1  Kapazitätsanteil
C2  Weiterer Kapazitätsanteil
7   Fuß
8   Schuh
9   Vorrichtung
10  Auswerteeinrichtung
11  Energiespeichereinrichtung
12  Datenübertragungseinrichtung
A   Ausschnitt

## Patentansprüche

1. Verfahren zur Bestimmung eines Untergrunds (4), wobei mindestens ein Kapazitätswert mindestens einer Elektrode (1) bestimmt wird, wobei die Elektrode (1) im Bereich einer Schuhsohle (2) eines Schuhs (8) angeordnet ist, wobei der Untergrund (4) in Abhängigkeit des Kapazitätswerts oder eines zeitlichen Verlaufs des Kapazitätswertes bestimmt wird, **dadurch gekennzeichnet, dass** der Kapazitätswert der Elektrode (1) bestimmt wird, der sich in Abhängigkeit einer Kapazität, die zwischen der Elektrode (1) und dem Untergrund (4) gebildet wird und/oder in Abhängigkeit einer Kapazität, die zwischen der Fußsohle (6) des Nutzers und der Elektrode (1) gebildet wird, ergibt, wobei der Kapazitätswert der Elektrode (1) bestimmt wird, indem diese zyklisch ge- und entladen wird, wobei ein untergrundspezifisches Gangbild eines Nutzers bestimmt wird, welches den Kapazitätswert oder dessen zeitlichen Verlauf umfasst, der in Abhängigkeit einer plantaren Druckverteilung oder eines zeitlichen Verlaufs der plantaren Druckverteilung erzeugt wird, wobei der Untergrund (4) in Abhängigkeit des Gangbilds bestimmt wird, wobei ein Grundkopplungsanteil der Kapazitätswerte bestimmt wird, wobei der Grundkopplungsanteil einen Anteil des Kapazitätswerts repräsentiert, der zwischen dem Untergrund (4) und der Elektrode (1) vorhanden ist, wobei der Grundkopplungsanteil in Abhängigkeit des Gleichanteils oder des Mittelwerts bestimmt wird, der in einem Zeitfenster in dem zeitlichen Verlauf des Kapazitätswerts bestimmt wird, wobei der Untergrund (4) in Abhängigkeit einer Zuordnung von Grundkopplungsanteilen zu Untergründen bestimmt wird.

2. Verfahren nach Anspruch 1, **dadurch gekennzeichnet, dass** ein spannungsführender Untergrund und/oder ein feuchter Untergrund in Abhängigkeit der Kapazitätswerte oder eines zeitlichen Verlaufs der Kapazitätswerte identifiziert wird.

3. Verfahren nach einem der vorangegangenen Ansprüche, **dadurch gekennzeichnet, dass** mehrere Elektroden (1) im Bereich der Schuhsohle (2) angeordnet sind, wobei Kapazitätswerte oder zeitliche Änderungen der Kapazitätswerte ausgewählter oder aller Elektroden (1) bestimmt werden, wobei das untergrundspezifische Gangbild in Abhängigkeit dieser Kapazitätswerte oder dieser zeitlichen Änderungen bestimmt wird.

4. Verfahren nach einem der vorangegangenen Ansprüche, **dadurch gekennzeichnet, dass** das untergrundspezifische Gangbild ein angelerntes Gangbild ist.

5. Verfahren nach einem der vorangegangenen Ansprüche, **dadurch gekennzeichnet, dass** die mindestens eine Elektrode (1) eine flächige Elektrode ist.

6. Verfahren nach einem der vorangegangenen Ansprüche, **dadurch gekennzeichnet, dass** in Abhängigkeit des bestimmten Untergrunds (4) mindestens eine Eigenschaft des Schuhs (8) eingestellt wird und/oder ein Warnsignal erzeugt wird.

**7.** Verfahren nach einem der vorangegangenen Ansprüche, **dadurch gekennzeichnet, dass** eine Haltung in Abhängigkeit der Kapazitätswerte oder eines zeitlichen Verlaufs des Kapazitätswerte bestimmt wird.

**8.** Vorrichtung zur Bestimmung eines Untergrunds (4), wobei die Vorrichtung mindestens einen Trägerkörper (5), mindestens eine Elektrode (1) und mindestens eine Auswerteeinrichtung (10) umfasst, wobei zumindest ein Teil der Vorrichtung im Bereich einer Schuhsohle (2) eines Schuhs (8) anordenbar ist, wobei die mindestens eine Elektrode (1) auf, an oder in dem Trägerkörper (5) angeordnet ist, wobei die Vorrichtung ausgebildet ist mindestens einen Kapazitätswert der mindestens einen Elektrode zu bestimmen und den Untergrund (4) in Abhängigkeit des Kapazitätswerts oder eines zeitlichen Verlaufs des Kapazitätswerts, **dadurch gekennzeichnet**, das sich, während der Verwendung, wenn die Vorrichtung im Bereich einer Schuhsohle (2) eines Schuhs (8) angeordnet ist, der Kapazitätswert der Elektrode (1) in Abhängigkeit einer Kapazität, die zwischen der Elektrode (1) und dem Untergrund (4) gebildet wird und/oder in Abhängigkeit einer Kapazität, die zwischen der Fußsohle (6) des Nutzers und der Elektrode (1) gebildet wird, ergibt, wobei die Vorrichtung eingerichtet ist, diesen Kapazitätswert der Elektrode (1) zu bestimmen, indem diese zyklisch ge- und entladen wird, wobei ein untergrundspezifisches Gangbild eines Nutzers bestimmt wird, welches die Kapazitätswerte oder deren zeitlichen Verläufe umfasst, die in Abhängigkeit einer plantaren Druckverteilung oder eines zeitlichen Verlaufs der plantaren Druckverteilung erzeugt werden, wobei der Untergrund (4) in Abhängigkeit des Gangbilds bestimmt wird, wobei ein Grundkopplungsanteil des Kapazitätswerts bestimmt wird, wobei der Grundkopplungsanteil einen Anteil des Kapazitätswerts repräsentiert, der zwischen dem Untergrund (4) und der Elektrode (1) vorhanden ist, wobei der Grundkopplungsanteil in Abhängigkeit des Gleichanteils oder des Mittelwerts bestimmt wird, der in einem Zeitfenster in dem zeitlichen Verlauf des Kapazitätswerts bestimmt wird, wobei der Untergrund (4) in Abhängigkeit einer Zuordnung von Grundkopplungsanteilen zu Untergründen bestimmt wird.

**9.** Vorrichtung nach Anspruch 8, **dadurch gekennzeichnet, dass** zumindest ein Teil der Vorrichtung als Einlegesohle ausgebildet ist.

**10.** Vorrichtung nach einem der Ansprüche 8 oder 9, **dadurch gekennzeichnet, dass** die Vorrichtung mindestens eine Isolations- bzw. Pufferschicht umfasst, wobei die Isolations- bzw. Pufferschicht über der mindestens einen Elektrode (1) angeordnet ist.

## Claims

**1.** Method for determining a ground surface (4), wherein at least one capacitance value of at least one electrode (1) is determined, the electrode (1) being arranged in the area of a shoe sole (2) of a shoe (8), wherein the ground surface (4) being determined as a function of the capacitance value or a time profile of the capacitance value, **characterized in that** the capacitance value of the electrode (1) is determined, which is a function of a capacitance that is formed between the electrode (1) and the ground surface (4) and / or as a function of a capacitance that is formed between the sole of the foot (6) of the user and the electrode (1), in which the capacitance value of the electrode (1) being determined by cyclically loading and unloading, an underground-specific gait pattern of a user being determined which corresponds to the Capacity value or its temporal profile, which is generated as a function of a plantar pressure distribution or a temporal profile of the plantar pressure distribution, in which the background (4) is determined depending on the gait pattern, as a basic coupling component of the capacitance values being determined, as the basic coupling component representing a component of the capacitance value that is present between the ground surface (4) and the electrode (1), in which the basic coupling component determining as a function of the direct component or the mean value which is determined in a time window in the course of the capacitance value over time, in which the surface (4) being determined as a function of an assignment of ground coupling components of surfaces.

**2.** Method according to claim 1, **characterized in that** a voltage carrying ground and / or a wet ground is identified as a function of the capacitance values or a time curve of the capacitance values.

**3.** Method according to any one of the preceding claims, **characterized in that** a plurality of electrodes (1) are arranged in the area of the shoe sole (2), in which the capacity values or changes in time of the capacity values of selected or all electrodes (1) being determined, whereby the underground-specific gait pattern is determined as a function of these capacity values or this change over time.

4. Method according to any one of the preceding claims, **characterized in that** the underground-specific gait is a learned/trained gait.

5. Method according to any one of the preceding claims, **characterized in that** the at least one electrode (1) is a flat electrode.

6. Method according to any one of the preceding claims, **characterized in that** at least one property of the shoe (8) is set and/or a warning signal is generated depending on the particular ground surface (4).

7. Method according to any one of the preceding claims, **characterized in that** a posture is determined depending on the capacity values or a time course of the capacity values.

8. Device for determining a ground surface (4), in which the device comprises at least one support body (5), at least one electrode (1) and at least one analysation device (10), at least part of the device in the area of a shoe sole (2) shoe (8) can be arranged, wherein at least one electrode (1) being arranged on, on or in the carrier body (5), wherein the device being designed to determine at least one capacitance value of the at least one electrode and the ground surface (4) as a function of the capacitance value or a time curve of the capacitance value, **characterized in that**, during use, when the device is arranged in the region of a shoe sole (2) of a shoe (8), the capacitance value of the electrode (1) varies as a function of a capacitance between the electrode (1) and the ground surface (4) is formed and/or as a function of a capacitance between the sole of the foot (6) of the user and the electrode (1) results, the device being set up to determine this capacitance value of the electrode (1) by cyclically loading and unloading it, whereby an underground-specific gait pattern of a user is determined, which comprises the capacitance values or their temporal profiles, which are generated as a function of a plantar pressure distribution or a time course of the plantar pressure distribution, wherein the ground surface (4) being determined as a function of the gait pattern, in which a ground coupling component of the capacity value being determined, wherein the ground coupling component representing a component of the capacity value, that is present between the ground surface (4) and the electrode (1), at which the basic coupling component being determined as a function of the constant component or the mean value, which is determined in a time window in the temporal course of the capacitance value, wherein the ground surface (4) depending on an assignment of ground coupling components to underground is determined.

9. Device according to claim 8, **characterized in that** at least part of the device is designed as an insole.

10. Device according to one of claims 8 or 9, **characterized in that** the device comprises at least one insulation or buffer layer, whereby the insulation or buffer layer is being arranged over the at least one electrode (1).

**Revendications**

1. Procédé de détermination du type de la surface du sol (4), dans laquelle au moins une valeur de capacité d'au moins une électrode (1) est déterminée, l'électrode (1) étant disposée dans la région d'une semelle de chaussure (2) d'une chaussure (8), surface du sol (4)) est déterminée en fonction de la valeur de capacité ou d'une évolution temporelle de la valeur de capacité, **caractérisée en ce que** la valeur de capacité de l'électrode (1) est déterminée, qui est fonction d'une capacité qui est formée entre l'électrode (1) et la surface du sol (4) et/ou en fonction d'une capacité qui se forme entre la plante du pied (6) de l'utilisateur et l'électrode (1), la valeur de capacité de l'électrode (1) étant déterminée en la chargeant et en la déchargeant cycliquement, le modèle de marche d'un utilisateur étant déterminé, qui comprend la valeur de capacité ou son profil temporel, qui comprend la valeur de capacité ou son évolution temporelle qui est générée en fonction d'une distribution de la pression plantaire ou d'une évolution temporelle de la distribution de la pression plantaire, le fond (4) étant déterminé en fonction du schéma de marche, une partie de couplage de base des valeurs de capacité étant déterminée, la partie de couplage de base représentant une partie de la valeur de capacité présente entre le fond (4) et l'électrode (1), dans laquelle la composante de couplage de base est déterminée en fonction de la composante directe ou de la valeur moyenne déterminée dans une fenêtre temporelle au cours de la valeur de capacité au cours du temps, dans le fond (4) étant déterminé en fonction d'une affectation de composants de couplage de base de la surface du sol.

2. Procédé selon la revendication 1, **caractérisé en ce qu'**un surface du sol vivant et/ou une surface de sol humide est identifié en fonction des valeurs de capacité ou d'une évolution temporelle des valeurs de capacité.

3. Procédé selon n'importe laquelle des revendications précédentes, **caractérisé en ce que** plusieurs électrodes (1) sont disposées dans la zone de la semelle de chaussure (2), les valeurs de capacité ou les variations dans le temps des valeurs de capacité des électrodes sélectionnées ou de toutes les électrodes (1) étant déterminées, le schéma de marche spécifique au sol en fonction des valeurs de capacité ou de ce changement dans le temps est déterminée.

4. Procédé selon n'importe laquelle des revendications précédentes, **caractérisé en ce que** le schéma de marche spécifique sur la surfaces du sol est un schéma de marche appris.

5. Procédé selon n'importe laquelle des revendications précédentes, **caractérisé en ce que** la au moins une électrode (1) est une électrode plate.

6. Procédé selon n'importe laquelle des revendications précédentes, **caractérisé en ce qu'**au moins une propriété de la chaussure (8) est réglée et/ou un signal d'avertissement est généré en fonction de la surface du sol particulière (4).

7. Procédé selon n'importe laquelle des revendications précédentes, **caractérisé en ce qu'**une posture est déterminée en fonction des valeurs de capacité ou d'une évolution temporelle des valeurs de capacité.

8. Dispositif de détermination du type de surface du sol (4), le dispositif comprenant au moins un corps de support (5), au moins une électrode (1) et au moins un dispositif d'analyse (10), au moins une partie du dispositif au niveau de la semelle de chaussure (2) chaussure(8) peut être agencé, avec au moins une électrode (1) disposée sur, ou dans le corps porteur (5), le dispositif étant conçu pour déterminer au moins une valeur de capacité d'au moins une électrode et de la surface du sol (4) en fonction de la valeur de capacité ou d'une évolution temporelle de la valeur de capacité, **caractérisé en ce que**, lors de l'utilisation, lorsque le dispositif est disposé dans la région d'une semelle de chaussure (2) d'une chaussure (8), la valeur de capacité de l'électrode (1) varie en fonction d'une capacité entre l'électrode (1) et surface du sol (4) formée et/ou en fonction d'une capacité entre la plante du pied (6) de l'utilisateur et l'électrode (1) résultant, le dispositif étant configuré pour déterminer cette valeur de capacité de l'électrode (1) en la chargeant et en la déchargeant de manière cyclique, moyennant la détermination d'un schéma de marche spécifique au sol d'un utilisateur est déterminé, qui comprend les valeurs de capacité ou leurs profils temporels, et qui sont générés en fonction d'une distribution de pression plantaire ou d'une évolution temporelle de la distribution de pression plantaire, dans lequel le sol (4) est déterminé en fonction du schéma de marche, dans lequel une partie de couplage de base de la valeur de capacité est déterminée, la partie de couplage de base représente une partie de la valeur de capacité entre les surface de sol (4) et l'électrode (1) sont présents, la composante de couplage de base étant déterminée en fonction de la composante constante ou de la valeur moyenne qui est déterminée dans une fenêtre temporelle dans le cours temporel de la valeur de capacité, en quoi le surface du sol (4) est déterminé en fonction d'une affectation de composants de couplage de base aux sols.

9. Dispositif selon la revendication 8, **caractérisé en ce qu'**au moins une partie du dispositif est conçue comme une semelle intérieure.

10. Dispositif selon l'une des revendications 8 ou 9, **caractérisé en ce que** le dispositif comprend au moins une couche isolante ou tampon, la couche isolante ou tampon étant disposée au-dessus de la au moins une électrode (1).

Fig.1

Fig.2

Fig.3

Fig.4

## IN DER BESCHREIBUNG AUFGEFÜHRTE DOKUMENTE

### In der Beschreibung aufgeführte Patentdokumente

- WO 2014032181 A
- KR 1020090036732 A
- US 20140135954 A1
- WO 2009089406 A2
- US 2005183292 A1

### In der Beschreibung aufgeführte Nicht-Patentliteratur

- Designing sensitive wearable capacitive sensors for activity recognition. **CHENG et al.** Sensors Journal. IEEE, Oktober 2013, vol. 13, 3935-3947
- Electric Field Imaging. **J. R. SMITH.** PhD Thesis. MIT - Massachusetts Institute of Technology, 1999